(19) Europäisches Patentamt
European Patent Office
Office européen des brevets

(11) **EP 2 465 571 B1**

(12) **EUROPÄISCHE PATENTSCHRIFT**

(45) Veröffentlichungstag und Bekanntmachung des Hinweises auf die Patenterteilung:
**18.01.2017 Patentblatt 2017/03**

(51) Int Cl.:
*A61N 1/05* (2006.01)      *A61N 1/08* (2006.01)

(21) Anmeldenummer: **11191741.5**

(22) Anmeldetag: **02.12.2011**

(54) **Implantierbares Gerät**

Implantable device

Appareil implantable

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.12.2010 US 424075 P**

(43) Veröffentlichungstag der Anmeldung:
**20.06.2012 Patentblatt 2012/25**

(73) Patentinhaber: **BIOTRONIK SE & Co. KG**
**12359 Berlin (DE)**

(72) Erfinder: **Büssing, Heinrich**
**10317 Berlin (DE)**

(74) Vertreter: **Galander, Marcus et al**
**Biotronik SE & Co. KG**
**Corporate Intellectual Properties**
**Woermannkehre 1**
**12359 Berlin (DE)**

(56) Entgegenhaltungen:
EP-A2- 1 923 095      WO-A1-2010/044019
WO-A1-2010/065049      WO-A2-2007/047966

• **BONMASSAR G: "Resistive Tapered Stripline (RTS) in Electroencephalogram Recordings During MRI", IEEE TRANSACTIONS ON MICROWAVE THEORY AND TECHNIQUES, IEEE SERVICE CENTER, PISCATAWAY, NJ, US, Bd. 52, Nr. 8, 1. August 2004 (2004-08-01) , Seiten 1992-1998, XP011115660, ISSN: 0018-9480, DOI: 10.1109/TMTT.2004.832688**

**Beschreibung**

Die Erfindung betrifft ein permanent oder temporär implantierbares Gerät mit einem langgestreckten elektrischen Leiter.

Solche Geräte, beispielsweise Elektrodenleitungen für die Elektrostimulation, haben den Nachteil, dass sich ihr elektrischer Leiter in einem Kernspintomografen erwärmen kann, weil die im Kernspintomografen herrschenden wechselnden Magnetfelder in dem elektrischen Leiter nicht unbeachtliche elektrische Ströme induzieren. Deshalb können Herzschrittmacherpatienten heutzutage in der Regel nicht oder nur eingeschränkt in einem Kernspintomografen untersucht werden.

An implantierbaren Herzschrittmachern oder Defibrillatoren sind nämlich typischerweise wenigstens eine Stimulationselektrodenleitung angeschlossen, die an ihrem proximalen, zum Anschluss an den Herzschrittmacher oder Defibrillator vorgesehenen Ende einen standardisierten elektrischen Anschluss aufweist und an ihrem distalen, zur Platzierung im Herzen vorgesehenen Ende einen oder mehrere Elektrodenpole aufweist. Ein solcher Elektrodenpol dient zur Abgabe elektrischer Impulse an das Gewebe (Myokard) des Herzens oder zum Abfühlen elektrischer Felder, um im Rahmen des sogenannten Sensings eine Aktivität eines Herzens abfühlen zu können. Elektrodenpole sind typischerweise in Form eines Rings um die Elektrodenleitung mit einer elektrisch leitenden Oberfläche oder in Form einer Spitzen- oder Tipelektrode am distalen Ende der Elektrodenleitung vorgesehen. Die Elektrodenpole sind über eine oder mehrere elektrische Leitungen mit Kontakten des elektrischen Anschlusses der Elektrodenleitung an deren proximalem Ende elektrisch leitend verbunden. Somit verlaufen zwischen den Kontakten des elektrischen Anschlusses die Elektrodenleitungen an deren proximalen Ende und den Elektrodenpolen am distalen Ende der Elektrodenleitung ein oder mehrere elektrische Leitungen, die einen oder mehrere der Elektrodenpole mit einem oder mehreren der Kontakte elektrisch verbinden. Diese elektrischen Leitungen können einerseits zur Übertragung von Stimulationsimpulsen zu den Elektrodenpolen oder zur Übertragung mittels der Elektrodenpole aufgenommener elektrischer Signale zum proximalen Ende der Elektrodenleitung genutzt werden und werden im Verlauf der weiteren Beschreibung als Funktionsleitung bezeichnet. Solche Funktionsleitungen sind für die Funktionen der jeweiligen Elektrodenleitung erforderliche elektrische Leiter und als solche der Gefahr ausgesetzt, dass in ihnen durch äußere Wechselmagnetfelder elektrische Ströme induziert werden, die beispielsweise zu einer unerwünschten Erwärmung der Funktionsleitungen oder der mit ihr verbundenen Elektrodenpole führen kann.

Aus der WO2007047966A2 sind Elektrodenleitungen bekannt, die auf Grund von Filtern, kapazitiven und induktiven Kopplungen auch in Umgebungen mit äußeren Wechselmagnetfeldem die elektrische Ströme induzieren verwendbar sind.

Der Erfindung liegt die Aufgabe zugrunde, ein Gerät zu schaffen, welches das zuvor beschriebene Problem löst.

Erfindungsgemäß wird diese Aufgabe durch ein temporär oder permanent implantierbares medizinisches Gerät mit mindestens einer langgestreckten elektrischen Leitung gelöst, die einen Funktionsleiter enthält, der durch ein Dielektrikum mit dem inneren Durchmesser $d_{iel}$ von umliegenden Material isoliert ist,

- der mit einem Elektrodenpol zur Abgabe von Therapiesignalen oder zur Erfassung von Diagnosesignalen verbunden ist, welcher im implantierten Zustand einen Lastwiderstand $Z_L$ für elektromagnetische Wellen darstellt,
- der zwischen seinem proximalen Ende und dem Elektrodenpol mindestens einen ersten Längsabschnitt eines ersten Leitungswellenwiderstandes $Z_0$ für elektromagnetische Radiofrequenz-Wellen aufweist, und
- der unmittelbar angrenzend an den ersten Längsabschnitt mindestens einen zweiten, mindestens 0,25 innere Durchmesser ($d_{iel}$) langen, im Vergleich mit dem ersten Längsabschnitt kürzeren Längsabschnitt eines zweiten Leitungswellenwiderstandes $Z_1$ für elektromagnetische Radiofrequenz-Wellen aufweist, wobei der zweite Leitungswellenwiderstand entweder größer oder kleiner als der erste Leitungswellenwiderstand und größer oder kleiner ist als der Lastwiderstand des Elektrodenpols, wobei der Funktionsleiter (26) einen Innenleiter und einen hohlzylindrischen Außenleiter aufweist, wobei der Innenleiter im zweiten Längsabschnitt (L2) eine vom Innenleiter im ersten Längsabschnitt (L1) abweichende geometrische Form hat, wobei der Innenleiter im ersten Längsschnitt (L1) holwendelförmig und im zweiten Längsabschnitt (L2) zylinderförmig ist, und wobei der zylinderförmige zweite Längsabschnitt (L2) des Innenleiters einen größeren Durchmesser als die Holwendel des Innenleiters im ersten Längsabschnitt (L1) aufweist.

Die elektrische Leitung des erfindungsgemäßen medizinischen Geräts enthält einen Funktionsleiter, welcher zwischen seinem proximalen Ende und einem verbundenen Elektrodenpol Leitungsabschnitte von vorzugsweise stark unterschiedlichem Wellenwiderstand hat. Diese dämpfen und transformieren einen Übergangswiderstand des verbundenen Elektrodenpols ins Gewebe derart, dass eine Fehlanpassung für Hochfrequenz erzielt werden kann und dadurch im implantierten Zustand im Falle einer Einwirkung von hochfrequenten magnetischen Wechselfeldern, wie sie in der Magnetresonanztomographie zum Einsatz kommen, die an das Gewebe übertragene Leistung gesenkt wird. Dadurch kann eine Erwärmung des Gewebes insbesondere nahe dem Elektrodenpol oder gegebenenfalls nahe den Elektrodenpolen deutlich reduziert werden.

Bei der elektrischen Leitung handelt es sich vorzugsweise um eine Elektrodenleitung für die Diagnose oder Therapie der Herzaktivität.

Der Unterschied zwischen dem ersten und zweiten Leitungswellenwiderstand ist in Ausführungsbeispielen des medizinischen Geräts so ausgeprägt, dass ein Realteil des zweiten Leitungswellenwiderstands für elektromagnetische Radiofrequenz-Wellen um einen Faktor von mindestens zwei kleiner ist als ein Realteil des ersten Leitungswellenwiderstands. Bevorzugt ist dieser Unterschied sogar größer und beträgt der genannte Faktor mindestens fünf, und in besonders bevorzugten Ausführungsbeispielen mindestens zehn.

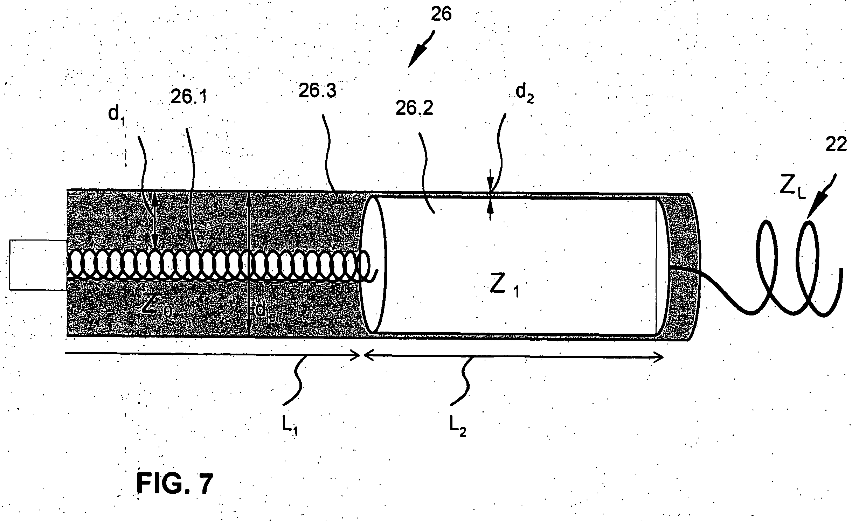

Untersuchungen der Erfinder haben gezeigt, dass der im Vergleich mit dem ersten Längsabschnitt kürzere zweite Längsabschnitt mindestens ein Viertel des inneren Dielektrikumdurchmessers ($d_{iel}$, in Figur 7) oder mindestens zehn Dielektrikumschichtdicken ($d_2$ in Figur 7) lang sein sollte, um die Wärmeübertragung zu reduzieren. Bis zu einer Länge des zweiten Längsabschnitts von einem Viertel des inneren Leitungsdielektrikumdurchmessers haben die Erfinder in Versuchen bei schwacher Dämpfung durch den Außenleiter (das Gewebe) sogar eine Erhöhung der Wirkleistung an einer im Versuch eingesetzten Spitzen (Tip)-Elektrode einer Elektrodenleitung festgestellt.

Die Wirksamkeit des zweiten Längsabschnitts für die Reduzierung der an das Gewebe übertragenen Wirkleistung steigt danach mit zunehmender Länge des zweiten Längsabschnitts. Besonders günstige Längen des zweiten Längsabschnitts liegen im Bereich bis maximal 5 $d_{iel}$. Vorzugsweise überschreitet der zweite Längsabschnitt eine Länge von etwa 10 $d_{iel}$, nicht. Die Wirksamkeit des zweiten Längenabschnitts hängt auch vom Grad der Dämpfung ab, welche Radiofrequenz-Wellen in der Leitung (engl.: transmission line) erfahren. Je höher die Dämpfung, desto geringer ist die Wirkleistung am Elektrodenpol.

Der Funktionsleiter weist einen Innenleiter und einen hohlzylindrischen Außenleiter auf, wobei der Innenleiter im zweiten Längsabschnitt eine vom Innenleiter im ersten Längsabschnitt abweichende geometrische Form hat. Der Innenleiter ist im ersten Längsabschnitt des elektrischen Leiters hohlwendelförmig und im zweiten Längsabschnitt zylinderförmig. Der zylinderförmige zweite Längsabschnitt des Innenleiters hat einen größeren Durchmesser als die Hohlwendel des Innenleiters im ersten Längsabschnitt. Vom Außenleiter sollte er jedoch durch ein inneres Dielektrikum getrennt, also elektrisch isoliert sein. Dieses innere Dielektrikum kann als Beschichtung oder Film realisiert sein. Nach Stand der Technik sind Schichtstärken bis herunter zu ca. 100nm oder sogar weniger möglich, z.B. durch PVD (plasma vapor deposition) oder CVD (chemical vapor deposition). Je dünner die Schichtdicke, desto stärker die Reduktion der ins Gewebe übertragenen Leistung. Jedoch ist eine sehr dünne Schicht anfälliger gegen mechanische Beanspruchung. Sie könnte z.B. abscheuern oder durch Kratzer ihre elektrische Isolationseigenschaft verlieren.

Bevorzugt wird als Kompromiss zwischen elektrischer Wirkung und mechanischer Stabilität eine Schichtdicke von 0,5 $\mu$m bis 2,5 $\mu$m, besonders bevorzugt von 1 $\mu$m.

Der Innenleiter kann alternativ im ersten Längsabschnitt als Seilzuleitung ausgeführt sein und im zweiten Abschnitt hohlwendelförmig.

Die Erfindung findet bei Elektrodenleitungen mit beliebigem Typ von Elektrodenpolen Anwendung. Der Elektrodenpol kann beispielsweise als Tip-Elektrodenpol oder als Ring-Elektrodenpol ausgeführt sein. Es können auch mehrere Elektrodenpole an einem Funktionsleiter vorgesehen sein. In manchen Ausführungsbeispielen schließt der Elektrodenpol unmittelbar an den zweiten Längsabschnitt des Funktionsleiters an. Dies ist herstellungstechnisch von Vorteil, weil der erste Längsabschnitt nicht unterbrochen werden muss. Es ist jedoch in einer Variante vorgesehen, den zweiten Längsabschnitt in den Funktionsleiter mit Abstand vom Elektrodenpol einzubetten, so dass der zweite Längsabschnitt in beiden Längsrichtungen von Leiterabschnitten vom Typ des ersten Längsabschnitts umgeben ist.

Nachfolgend werden das medizinische Gerät der vorliegenden Erfindung und einige Ausführungsbeispiele unter Bezugnahme auf die Figuren näher erläutert.

Es zeigen:

Fig. 1      Ausführungsbeispiele medizinischer Geräte in Form eines Herzschrittmachers und einer daran angeschlossenen Elektrodenleitung;

Fig. 2      ein Ersatzschaltbild eines mit einem Elektrodenpol verbundenen Funktionsleiters einer Elektrodenleitung nach dem Stand der Technik;

Fig. 3      ein Ersatzschaltbild eines mit einem Elektrodenpol verbundenen Funktionsleiters einer Elektrodenleitung nach einem Ausführungsbeispiel der vorliegenden Erfindung;

Fig. 4      in einem schematischen Ersatzschaltbild Ersatzwiderstände zwischen den Elektrodenpolen Spitze und Ring einer Elektrodenleitung und ihrer Gegenelektrode in einer Sternschaltung;

Fig. 5      in einem schematischen Ersatzschaltbild Ersatzwiderstände zwischen den Elektrodenpolen Spitze und Ring einer Elektrodenleitung und ihrer Gegenelektrode in einer Dreieckschaltung;

Fig. 6    drei Konstellationen, in denen jeweils ein Gesamtwiderstand zwischen zwei Punkten A und B gemessen wird, um Teilwiderstände RT, RR und RG der Ersatzschaltbilder aus Figur4 und Figur5 für unterschiedliche Frequenzen zu errechnen;

Fig. 7    eine schematische Darstellung eines Beispiels eines Funktionsleiters einer Elektrodenleitung;

Fig. 8 und 9    je eine graphische Darstellung des Ergebnisses einer Simulationsrechnung, welche elektromagnetische Energie sowie magnetische Feldlinien um einen Längsabschnitt von 0 bis 1 Meter eines langgestreckten elektrischen Leiters gemäß dem Stand der Technik beziehungsweise gemäß einem Ausführungsbeispiel der vorliegenden Erfindung wiedergibt; und

Fig. 10 bis 12    Diagramme von Leistungsparametern für ein Ausführungsbeispiel, bei dem zwischen einem Elektrodenpol in Form einer Elektrodenspitze und der Funktionsleitung ein kurzes, schwach bedämpftes Stück Leitung eingebaut ist.

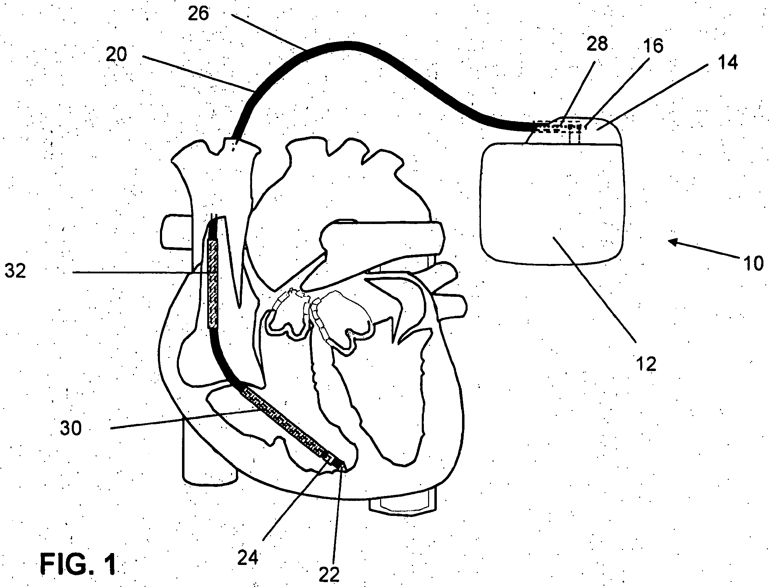

Figur 1 zeigt als Beispiele implantierbarer medizinischer Geräte einen implantierbaren Herzstimulator 10 und eine daran angeschlossene implantierbare Elektrodenleitung 20.

Der implantierbare Herzstimulator 10 kann ein Herzschrittmacher oder ein Kardioverter/Defibrillator (ICD) sein. Im dargestellten Ausführungsbeispiel ist der Herzstimulator 10 ein ventrikulärer Herzschrittmacher und Defibrillator. Andere bekannte Herzstimulatoren sind Zweikammerherzschrittmacher zur Stimulation des rechten Atriums und des rechten Ventrikels oder biventrikuläre Herzschrittmacher, die zusätzlich zum rechten Ventrikel auch den linken Ventrikel stimulieren können.

Derartige Stimulatoren besitzen typischerweise ein Gehäuse 12, das im Regelfall aus Metall besteht und somit elektrisch leitend ist und als großflächiger Elektrodenpol dienen kann. An der Außenseite des Gehäuses 12 ist typischerweise ein Anschlussgehäuse 14 befestigt, das auch als Header bezeichnet wird. Ein derartiger Header weist typischerweise Kontaktbuchsen zur Aufnahme von Steckkontakten auf. Die Kontaktbuchsen besitzen elektrische Kontakte 16, die über entsprechende Leiter mit einer in dem Gehäuse 12 des Herzstimulators 10 angeordneten Elektronik verbunden sind.

Die Elektrodenleitung 20 stellt im Sinne dieser Erfindung ebenfalls ein implantierbares medizinisches Gerät dar. Am distalen Ende der Elektrodenleitung 20 sind in an sich bekannter Manier Elektrodenpole in Form einer Spitzen- oder Tip-Elektrode 22 und einer in deren Nähe angeordneten Ringelektrode 24 angeordnet. Die Elektrodenpole 22 und 24 sind so ausgebildet, dass sie je nach Funktion eines Herzstimulators, an den die Elektrodenleitung 20 angeschlossen ist, zum Abfühlen elektrischer Potenziale des Herzgewebes (Myokards) dienen oder zur Abgabe elektrischer Signale, beispielsweise zur Abgabe von Stimulationsimpulsen an das sie umgebende Herzgewebe, ausgebildet sind. Figur 1 zeigt, wie sich die Elektrodenpole, also die Tip-Elektrode 22 und die Ringelektrode 24, im Anwendungsfall die Elektrodenleitung 20, im Apex eines rechten Ventrikels eines Herzens befinden.

Sowohl die Tip-Elektrode 22 als auch die Ringelektrode 24 sind über jeweils wenigstens einen elektrischen Leiter 26 mit einem Steckkontakt 28 am proximalen Ende der Elektrodenleitung 20 elektrisch verbunden. Der Steckkontakt 28 besitzt elektrische Kontakte, die mit den elektrischen Kontakten 16 der Kontaktbuchse im Anschlussgehäuse 14 des implantierbaren Herzstimulators korrespondieren.

Wie weiter unten näher erläutert wird, können die elektrischen Leiter 26 in der Elektrodenleitung 20 in unterschiedlichen Längsabschnitten als annähernd gestreckte Seilzuleiter oder als helixförmig gewendelte Leiter ausgebildet sein. Solche Leiter, die funktionale Elektrodenpole mit elektrischen Kontakten des Steckkontaktes am proximalen Ende der Elektrodenleitung 20 elektrisch leitend verbinden, werden im Rahmen dieses Textes auch als Funktionsleiter bezeichnet, da sie beispielsweise der Therapie dienende elektrische Signale von Steckkontakt zum jeweiligen Elektrodenpol übertragen oder abgefühlte elektrische Potentiale repräsentierende Signale vom jeweiligen Elektrodenpol zum Steckkontakt führen und somit der elementaren Funktion des medizinischen Gerätes dienen.

Die elektrischen Funktionsleiter 26, die die Elektrodenpole 22 bzw. 24 mit den elektrischen Kontakten des Steckers 28 der Elektrodenleitung 20 verbinden, sind über den größten Teil ihrer Länge von einer isolierenden Hülle umgeben, so dass ein elektrischer Kontakt zum Gewebe des Herzens gezielt über die Elektrodenpole zustande kommt.

Neben den Elektrodenpolen 22 und 24, die typischerweise der (in diesem Fall ventrikulären) Stimulation des Herzgewebes dienen, weist die Elektrodenleitung 20 auch noch zwei großflächigere Elektrodenpole 30 und 32 auf, die als Defibrillationselektroden dienen und von wenigstens einem blank liegenden helixartig gewendelten Draht gebildet sind.

Es sei darauf hingewiesen, dass die Erfindung im Rahmen dieses Ausführungsbeispiels anhand eines rechtsventrikulären Herzschrittmachers und Defibrillators erläutert wird. Als medizinisches Gerät im Sinne der Erfindung kann grundsätzlich aber beispielsweise auch eine Ablationselektrodenleitung dienen, die im Anwendungsfall ebenfalls bis ins Herz eines Patienten hineinragt und die von einem außerhalb des Patienten angeordneten Gerät gesteuert wird und hierzu mit diesem verbunden ist. Weiterhin können solche Elektrodenleitungen unter fachüblicher Anpassung an die speziellen Erfordernisse des jeweiligen Anwendungsgebiets auch zur Stimulation und Ableitung von Signalen an Nerven, Gehirn, und anderen Organen oder für die Zuleitung von implantierbaren Sensoren Verwendung finden.

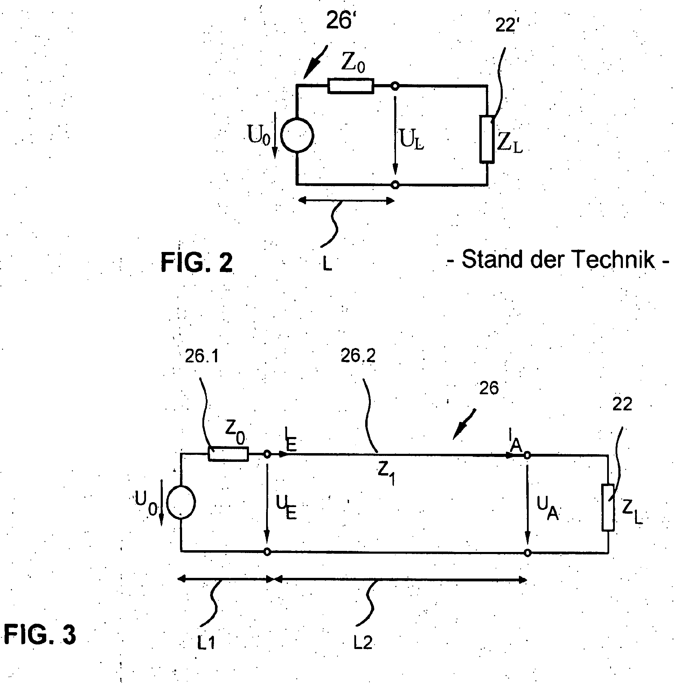

Figur 2 zeigt ein Ersatzschaltbild eines Funktionsleiters einer Elektrodenleitung nach dem Stand der Technik. Der Funktionsleiter 26' bildet mit seiner Längserstreckung L einen ersten Leitungswellenwiderstand $Z_0$. Der Elektrodenpol 22' bildet einen Last-Leitungswellenwiderstand $Z_L$. Am proximalen Ende des Funktionsleiters, nachfolgend auch als Anfang des Funktionsleiters bezeichnet, ist eine Spannung $U_0$ zugeführt. Über einem Elektrodenpol 22' fällt am distalen Ende des Funktionsleiters eine Spannung $U_L$ ab.

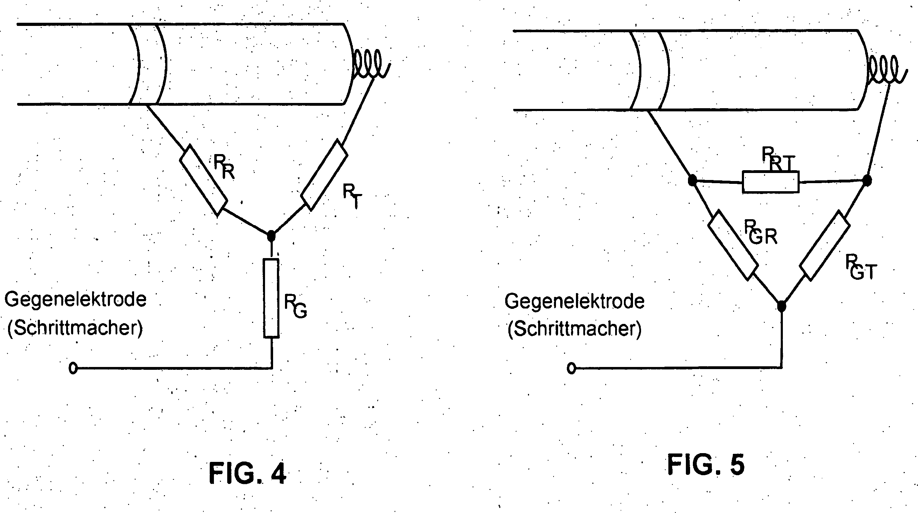

Der von einer Elektrodenspitze gebildete Elektrodenpol 22' ist, wie weiter unten anhand der Figuren 4 bis 6 erläutert wird, beispielsweise durch einen komplexen Last-Leitungswellenwiderstand von

$$Z_L = 233 - j138\,\Omega$$

gegen das Körpergewebe darstellbar.

Wenn nun der Funktionsleiter jedoch anders als in Figur 2 über seine Längserstreckung einen ersten Längsabschnitt L1 und einen davon verschiedenen zweiten Längsabschnitt L2, in Form eines Leiterstücks der Länge 1 mit einem vom ersten Leitungswellenwiderstand $Z_0$ und vom Last-Leitungswellenwiderstand $Z_L$ verschiedenen zweiten Leitungswellenwiderstand $Z_1$ enthält, das den Last-Leitungswellenwiderstand $Z_L$ auf $\check{Z}_L$ transformiert, ändern sich die Verhältnisse wesentlich. Das Ersatzschaltbild eines solchen erfindungsgemäßen Funktionsleiters 26 zeigt Figur 3.

Der Funktionsleiter 26 hat nun die zwei Längsabschnitte L1 und L2. Ihr Längenverhältnis zu einander ist in Figur 3 nicht dem tatsächlichen Verhältnis entsprechend dargestellt. Tatsächlich ist der erste Längsabschnitt L1 mit einem Funktionsleiterstück 26.1 wesentlich länger als der zweite Längsabschnitt L2 mit einem Funktionsleiterstück 26.2. Der zweite Längsabschnitt L2 hat eine Länge 1 und einen Leitungswellenwiderstand $Z_1$.

Zum proximalen Ende des Funktionsleiters 26 hin, am Anfang des zweiten Längsabschnitts, liegt eine Spannung $U_E$ an, und am distalen Ende des Funktionsleiters 26, im vorliegenden Beispiel zugleich auch das Ende des zweiten Längsabschnitts L2, liegt eine Spannung $U_A$ über dem Last-Leitungswellenwiderstand des Elektrodenpols 22 an.

Die Spannungen und Ströme, $U_E$, $I_E$, $U_A$ und $I_A$, am Anfang und Ende des zweiten Längsabschnitts L2 lassen sich in eine hinlaufende Welle und eine rücklaufende Welle aufteilen:

$$U_E = U_H\, e^{jkl} + U_R\, e^{-jkl} \qquad (1.a)$$

$$I_E = \frac{1}{Z_1}\left(U_H\, e^{jkl} - U_R\, e^{-jkl}\right) \qquad (1.b)$$

$$U_A = U_H + U_R \qquad (1.c)$$

$$I_A = \frac{1}{Z_1}\left(U_H - U_R\right) \qquad (1.d)$$

Man berechnet den Last-Leitungswellenwiderstand $Z_L$ und einen Transformierten Last-Leitungswellenwiderstand $\check{Z}_L$ aus den hin- und rücklaufenden Wellen wie folgt:

$$Z_L = Z_1\, \frac{U_H + U_R}{U_H - U_R} \qquad (2.a)$$

$$\widetilde{Z}_L = Z_1 \frac{U_H e^{jkl} + U_R e^{-jkl}}{U_H e^{jkl} - U_R e^{-jkl}} \qquad (2.b)$$

Eine Umformung ergibt für den transformierten Last-Leitungswellenwiderstand

$$\widetilde{Z}_L = \frac{Z_L \cos(kl) + jZ_1 \sin(kl)}{jZ_L \sin(kl) + Z_1 \cos(kl)} \qquad (3)$$

Die Leistung wird mit

$$P = U \cdot I^* \qquad (4)$$

berechnet.

Mit einem hochgestellten Stern versehene Größensymbole kennzeichnen das konjugiert Komplexe einer jeweiligen Zahl.

Somit kann die Leistung, die in den zweiten Längsabschnitt hineinfließt wie folgt berechnet werden:

$$P_E = U_E \cdot I_E^* = U_0 \frac{\widetilde{Z}_L}{\widetilde{Z}_L + Z_0} \cdot U_0^* \frac{1}{(\widetilde{Z}_L + Z_0)^*} \qquad (5)$$

$$P_E = U_E \cdot I_E^* = |U_0|^2 \frac{\widetilde{Z}_L}{|\widetilde{Z}_L + Z_0|^2} \qquad (6)$$

Die Leistung am Last-Leitungswellenwiderstand, also in der Elektrodenspitze, kann aus der hin- und rücklaufenden Welle berechnet werden.

$$P_L = (U_H + U_R) \cdot \frac{(U_H + U_R)^*}{Z_L^*} = \frac{|U_H + U_R|^2}{Z_L^*} \qquad (7)$$

Mit

$$U_E = \widetilde{Z}_L I_E \qquad (8)$$

leitet man aus Gleichung (1.b)

$$\frac{Z_1}{\widetilde{Z}_L} U_E = Z_1 I_E = U_H e^{jkl} - U_R e^{-jkl} \qquad (9)$$

ab und erhält somit durch Einsetzen und Umstellen der Gleichungen (1.a) und (1.b)

$$U_H = \frac{1}{2} \left( 1 + \frac{\widetilde{Z}_L}{Z_1} \right) U_E \, e^{-jkl} \qquad (10a)$$

$$U_R = \frac{1}{2}\left(1 - \frac{\widetilde{Z}_L}{Z_1}\right)U_E \, e^{jkl} \qquad\qquad (10b)$$

$$P_L = \frac{1}{Z_L^*}\left|\left(1 + \frac{\widetilde{Z}_L}{Z_1}\right)\cdot e^{-jkl} + \left(1 - \frac{\widetilde{Z}_L}{Z_1}\right)\cdot e^{jkl}\right|\cdot\frac{1}{4}U_E^2 = \frac{U_E^2}{Z_L^*}\left|\cos(jkl) - j\frac{\widetilde{Z}_L}{Z_1}\sin(jkl)\right| \qquad (11)$$

Es folgt anhand der Figuren 4, 5 und 6 eine Erläuterung zur Ermittlung der zugrunde zu legenden frequenzabhängigen Wellenleitungswiderstandswerte.

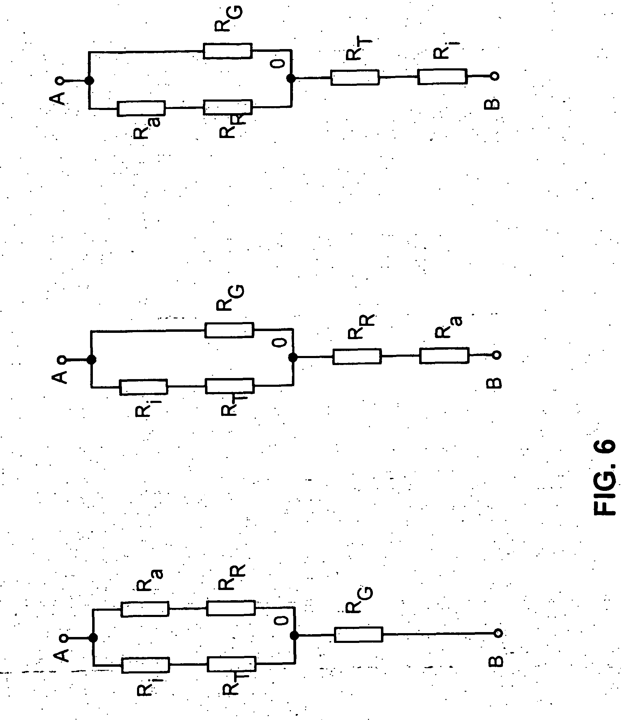

Figur 4 und 5 zeigen in einem schematischen Ersatzschaltbild Ersatzwiderstände zwischen den Elektrodenpolen Spitze (Tip) 22 und Ring 24 einer Elektrodenleitung 22 und ihrer Gegenelektrode in einer Sternschaltung (Figur 4) und Dreieckschaltung (Figur 5). Figur 6 zeigt diejenigen drei Konstellationen, in denen jeweils ein Gesamtwiderstand zwischen zwei Punkten A und B gemessen wird. Aus den Gesamtwiderständen dieser drei Netzwerke lassen sich für jede Frequenz, bei der gemessen wird, jeweils die Teilwiderstände RT, RR und RG errechnen.

Es wird ein Widerstand der Innenwendel Ri, der mit 37,6 Ω, und ein Widerstand der Außenwendel Ra, der mit 106,7 Ω bei jeder der verwendeten Frequenzen gemessen wurde, für die Berechnung der Widerstände der Elektrodenpole berücksichtigt werden. Der Leitungswiderstand der Gegenelektrode wird mit 0 Ω veranschlagt. Ri und Ra sind getrennt gemessen und bekannt.

Es wurden zwei verschiedene Elektrodenleitungen vermessen, die in ein Glas physiologischer Kochsalzlösung mit einer Gegenelektrode aus Platin-Iridium-Legierung gehalten wurden. Um zunächst die Induktivität, Kapazität und Widerstand der Zuleitungen zu isolieren, wurde bei verschiedenen Frequenzen mit einem LCR-Meter, Type: Fluke PM6306, die Impedanz zwischen Innen- und Außenleiter einer Elektrodenleitung mit der Spitze entweder in der Lösung oder freihängend in der Luft gemessen.

Die von den Erfindern in einer Versuchsreihe erzielten Messergebnisse sind nachfolgend in Tabelle 1 dargestellt. In jeder Zelle der Tabelle sind zwei Werte untereinander aufgeführt, die an zwei unterschiedlichen Elektrodenleitungen (oberer Wert BIOTRONIK Setrox S60, unterer Wert BIOTRONIK Setrox S45) gemessen wurden.

Tabelle 1

| Frequenz | Tip gegen Ring mit Kopf in der Lösung | | Tip gegen Ring mit Kopf in der Luft | |
|---|---|---|---|---|
| | R/Ω | C/nF | R/Ω | C/nF |
| 1MHz | 275,7 | 64 | 58 | 75,3 |
| | 222,2 | Nicht messbar | 34,9 | 59,8 |
| 500kHz | 277,8 | 340 | 55,2 | 83,3 |
| | 222,7 | 0,13 | 38,0 | 68,9 |
| 200kHz | 278,4 | 514 | 57,06 | 83,23 |
| | 223,8 | 412 | 38,15 | 67,7 |
| 100kHz | 278,87 | 721,4 | 56,65 | 83,18 |
| | 224,22 | 523 | 40,15 | 67,68 |
| 50kHz | 279,74 | 1029 | 66,24 | 83,19 |
| | 225,37 | 641 | 51,70 | 67,73 |
| 20kHz | 281,3 | 1630 | 115,8 | 83,22 |
| | 227,8 | 890 | Nicht messbar | 67,74 |

Die Messwerte der Tabelle lassen erkennen, dass ab Frequenzen von 500kHz die Induktivität der Leitung bei Messung mit offenem Kopf schon eine Rolle spielt. Aus den Messungen mit Kopf in der Luft (Unterbrechung zwischen Innen- und Außenleiter, bzw. zwischen Tip und Ring) ließ sich ein Wert des Kapazitätsbelages zwischen Innen- und Außenwendel zu 140 bis 150pF/m bestimmen.

Den Widerstandsbelag von Innen- und Außenwendel kann man mit Kopf im Wasser bei Frequenzen unter 500kHz bestimmen, wenn man den Widerstand zwischen Tip und Ring abzieht. Man sieht bei der Messung mit Kopf in der Salzlösung, dass bei höherer Frequenz die Induktivität der Leitung sich immer mehr auf das Messergebnis auswirkt und je nach Elektrodentyp, hier bei der Setrox S40, ab 1MHz Frequenz die Kapazität der Leitung sogar schon ausgleichen kann.

Bei niedrigen Frequenzen um 20kHz zeigt sich als kapazitiver Widerstand die Faraday-Kapazität der beiden Elektroden in Tabelle stark unterschiedlich und viel zu gering. Das wird auf Schäden an der fraktalen Struktur von Tip und Ring durch Einspannen der Elektrodenspitzen bei vorangegangenen Messungen zurückgeführt. Die Messwerte ab 50kHz Frequenz werden davon aber fast nicht mehr beeinflusst.

Es hat sich gezeigt, dass die gemessenen Widerstandswerte von der Position des Elektrodenkopfes zur Gegenelektrode unbeeinflusst bleiben, wenn die beiden weiter als ca. 4 Zentimeter voneinander entfernt sind. Das lässt schließen, dass die Widerstände RT, RR und RG aus Figur 4 die Widerstände des jeweiligen Elektrodenpols gegen einen unendlich fernen Punkt mit Nullpotenzial sind.

Die Widerstände der Elektroden untereinander wurden bei der Elektrodenleitung BIOTRONIK Setrox S60 wie folgt gemessen

Tabelle 2

| Frequenz | Tip und Ring gegen Gegenelektrode | Ring und Gegenelektr. Gegen Tip | Tip und Gegenelektr. gegen Ring |
|---|---|---|---|
| 1MHz | 77,7Ω<br>1,64µH | 137,6Ω<br>70nF | 156Ω<br>3,26µH |
| 500kHz | 77,8Ω<br>2,5µH | 137,8Ω<br>0,22µH | 155,4Ω<br>3,98µH |
| 200kHz | 77,9Ω<br>3,0µH | 138,04Ω<br>1,44µF | Nicht gemessen |
| 100kHz | 78,2Ω<br>Nicht messbar | 138,48Ω<br>1,222µF | 154,77Ω<br>3,41µH |
| 50kHz | 78,538Ω<br>Nicht messbar | 139,22Ω<br>1,5µF | 154,86Ω<br>3,49µH |
| 20kHz | 79,026Ω<br>9,11µF | 140Ω<br>2,3µF | 155,20Ω<br>28,27µF |

Es ergaben sich für die Widerstände von Tip, Ring und Gegenelektrode bei verschiedenen Frequenzen die in der nachfolgenden Tabelle 3 zusammengestellten Werte. Der Durchschnittswert (∅) ist fett gedruckt in der unteren Zeile eingetragen, dabei wurden für RT und RR der Mittelwert und für RG der Median veranschlagt.

| Frequenz | RT | RR | RG |
|---|---|---|---|
| 1MHz | 91,88Ω | 41,15Ω | 8,70Ω |
| 500kHz | 91,81Ω | 40,28Ω | 9,01Ω |
| 100kHz | 91,94Ω | 39,11Ω | 9,63Ω |
| 50kHz | 92,51Ω | 39,02Ω | 9,83Ω |
| 20kHz | 92,99Ω | 39,06Ω | 10,17Ω |
| ∅ | **92,2Ω** | **39,7Ω** | **9,6Ω** |

Die Leitfähigkeit des Elektrolyts ist auf $\sigma$=1,57 ... 1,67 S/m eingestellt und seine Permittivität ist $\varepsilon$=80.8,85·10-12 As/Vm so lässt sich mit der Formel

$$C = \frac{1}{R} \cdot \frac{\varepsilon}{\sigma}$$

hochrechnen, welche Kapazität zu den Widerständen jeweils parallel geschaltet gedacht werden muss. Sie ist in der Tabelle zusammen mit dem kapazitiven Blindwiderstand bei 64MHz aufgelistet. Die Messwerte für Tip und Ring schwanken um 1,2% (Tip), bzw. 2,7% (Ring), die Toleranz in der Leitfähigkeit der physiologischen Kochsalzlösung schwankt um 6,2%, so dass mit Abweichungen von 10% nach oben oder unten gerechnet werden muss.

|  | Tip Ring | | Gegenelektrode |
|---|---|---|---|
| **Kapazität** | | | |
| C | 4,54pF ... 4,95pF | 10,4pF ... 11,7pF | 37,4pF ... 54,2pF |
| $\varnothing$ | 4,74pF | 11,0pF | 45,5pF |
| **kapazitiver Blindwiderstand bei 64MHz** (Larmor-Frequenz bei MRI in 1,5T-Geräten) | | | |
| XC 64MHz | -j503$\Omega$ ... -j547$\Omega$ | -j212$\Omega$ ... -238$\Omega$ | -j45$\Omega$ ... -j65$\Omega$ |
| $\varnothing$ | -j524$\Omega$ | -j226$\Omega$ | -j55$\Omega$ |
| **Widerstand umgerechnet auf Kochsalzlösung nach ASTM** ($\sigma$=0,474 S/m) | | | |
| R $\varnothing$ | 315$\Omega$ | 136$\Omega$ | 33$\Omega$ |
| **Widerstand umgerechnet auf eine 0,03 molare Lösung** ($\sigma$=0,310 ... 0,370 S/m) | | | |
| R $\varnothing$ | 440$\Omega$ | 190$\Omega$ | 46$\Omega$ |

Eine Kochsalzlösung mit $\sigma$=0,474 S/m liefert die richtigen Ergebnisse für die Messung der Erwärmung nach der ASTM Norm (American Standard for Testing and Material), während eine 0,03 molare Kochsalzlösung die richtigen Werte für den Widerstand von Tip und Ring für Sensing und Pacing im Gewebe liefert.

Figur 7 ist eine schematische Darstellung eines Beispiels eines Funktionsleiters einer Elektrodenleitung. Das Ausführungsbeispiel lässt sich auch durch das Ersatzschaltbild der Figur 3 darstellen. Daher werden in Figur 7 weitgehend identische Bezugszeichen wie in Figur 3 verwendet. Der Funktionsleiter 26 hat einen hier nicht in voller Länge dargestellten ersten Längsabschnitt L1, in dem sein Innenleiter als Hohlwendel 26.1 ausgeführt ist und einen Leitungswellenwiderstand $Z_0$ bildet. Der Innenleiter 26.1 ist in diesem ersten Längsabschnitt L1 von einer Isolierung 26.3 umgeben, beispielsweise einer Silikonisolierung mit einer relativen Permittivität von $\varepsilon_r$ = 3 sowie einer verschwindenden elektrischen Leitfähigkeit $\sigma$ = 0$S$/m.

Im zum ersten Längsabschnitt L1 vergleichsweise sehr kurzen zweiten Längsabschnitt L2 des Funktionsleiters 26 ist der Innenleiter als zylindrischer Leiter 26.2 von beispielsweise 2 Millimeter Durchmesser und 10 Millimeter Länge gebildet, der mit einer Isolationsschicht, einem inneren Dielektrikum (z.B. einer Lackschicht) einer Stärke d2 von 10 Mikrometer umgeben ist. Dieser zylindrische Leiter 26.2 weist einen Leitungswellenwiderstand für elektromagnetische Radiofrequenzwellen von $Z1=(13,16+j6,67)\Omega$ auf.

Zum Vergleich: Im ersten Längsabschnitt L1 hat die Hohlwendel 26.1 einen deutlich höheren Leitungswellenwiderstand für elektromagnetische Radiofrequenzwellen, wie sich aus den oben im Zusammenhang mit den Figuren 4 bis 6 dargestellten Berechnungen und Messungen ergibt. Der entsprechende Leitungswellenwiderstand des Elektrodenpols 22 beträgt (bei $\varepsilon_r$ = 80, $\sigma$ = 0,47$S$ /m für Körpergewebe), wie erwähnt beispielsweise $Z_L$=233-j138$\Omega$ und liegt also ebenso deutlich über dem des Innenleiterstücks 26.2 im zweiten Längsabschnitt L2.

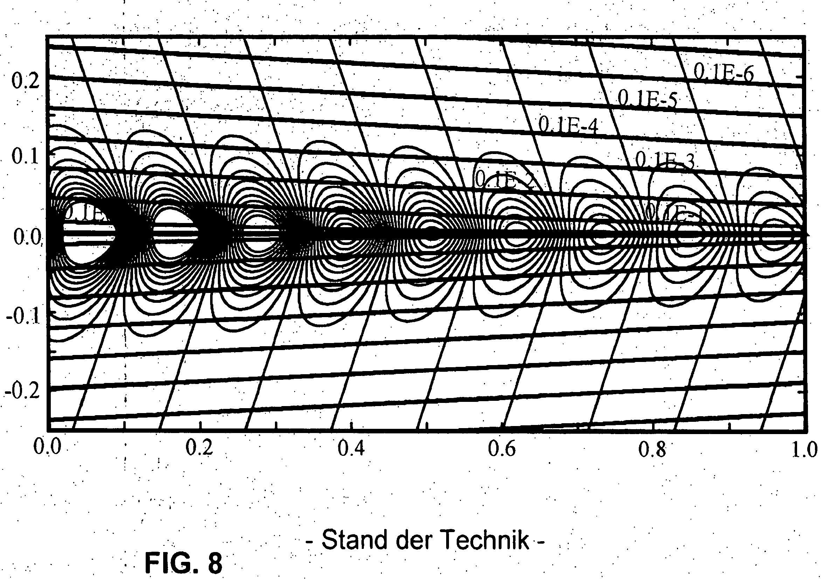

Figur 8 zeigt eine graphische Darstellung des Ergebnisses einer Simulationsrechnung, welche die elektrische Feldstärke sowie magnetische Feldlinien bzw. Umrisslinien von magnetischen Flussröhren um einen Längsabschnitt von 0 bis 1 Meter eines langgestreckten elektrischen Leiters gemäß dem Stand der Technik wiedergibt, der dem Ersatzschaltbild der Figur 2 entspricht und einen inneren Elektrolytdurchmesser $d_{iel}$ von 2mm hat..

Die dünn gezeichneten Linien sind die Feldlinien bzw. Flussröhren. Sie zeigen den Verlauf des Feldes, das sich um die sich auf der Abszisse des Diagramms erstreckende zylindrische Leitung ausbildet, wenn sich eine Hochfrequenzwelle in der Zeichnungsebene von links nach rechts entlang der Leitung ausbreitet. Auf der Ordinate ist der Abstand von der Abszisse in Metern zwischen -0,25 und 0,25 Metern aufgetragen. Negative Abstandswerte sind als Abstand in entgegengesetzter Richtung zur Richtung positiver Abstandswerte zu verstehen. Die fett gezeichneten Linien sind Isolinien und kodieren die elektromagnetische Energie, die sich in den betreffenden Radiuselementen $2\pi \rho\, d\rho$ befindet.

Die elektromagnetische Energie ist als über die Zeit gemittelte Energie zu verstehen, so dass sie nicht an den Stellen am größten ist, wo die Feldlinien dicht beieinander liegen, sondern generell dicht an der Leitung.

Zwischen zwei Isolinien nimmt der Energiewert logarithmisch um Faktor zehn ab. Daraus, dass die Feldlinienknäule, nach rechts hin kleiner werden, bzw. die Isolinien nach rechts hin konisch zulaufen, erkennt man, dass das Feld nach rechts hin abnimmt, die Leitung also bedämpft ist.

Die Figur zeigt das Feldbild um eine Elektrode nach dem Stand der Technik im Körpergewebe mit der Leitfähigkeit $\sigma$=4,74S/m und der relativen Permittivität $\varepsilon_r$=80, auf der sich eine Welle mit 64MHz ausbreitet. Der komplexwertige Wellenwiderstand ist in diesem Beispiel

$$Z_0=162,5+j12,34\Omega.$$

Die Elektrodenspitze ist durch einen komplexen Widerstand von $Z_L$=233-j138$\Omega$ gegen das Körpergewebe darstellbar. Der Lastwiderstand unterscheidet sich in seiner Größe nicht allzu viel vom Innenwiderstand, so dass in diesem Beispiel 61% der maximal möglichen Wirkleistung umgesetzt wird.

Die magnetischen Feldlinien kringeln sich zylinderrund um die Leitung herum und stehen an jeder Stelle genau senkrecht auf den elektrischen Feldlinien. Es handelt sich hier also um die Ausbreitung einer transversalen elektromagnetischen (TEM-) Welle. Man erkennt, dass ein isolierter Draht wie eine Elektrodenleitung im Körpergewebe eine Leitung darstellt, auf der sich eine TEM-Welle ausbreiten kann. Diese Leitung hat einen Wellenwiderstand, eine Dämpfung und eine Wellenlänge, die von der Frequenz abhängig sind. Die Länge einer dieser Zwiebeln ist eine halbe Wellenlänge. Man kann aus dem Diagramm die zu erwartende Wellenlänge von 23cm herauslesen.

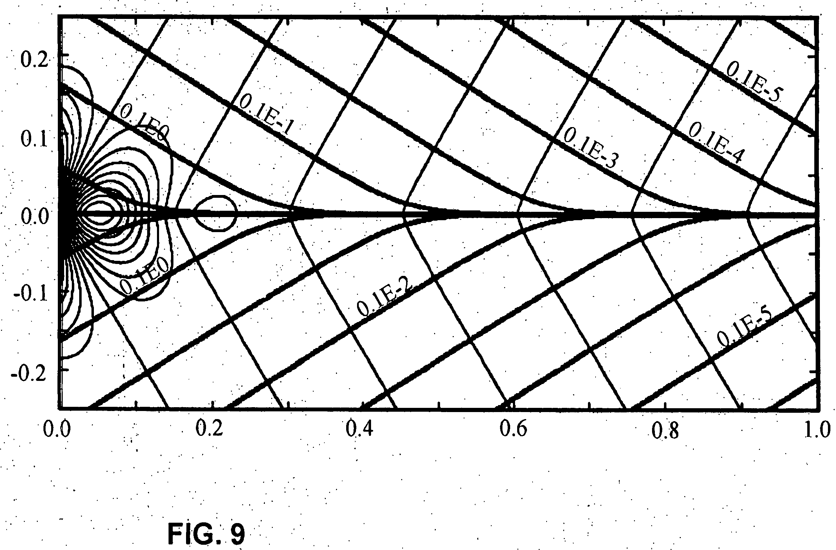

Wenn nun jedoch ein Stück Leitung der Länge 1 mit einem von $Z_0$ und $Z_L$ verschiedenen Leitungswellenwiderstand zwischengeschaltet wird, das den Lastwiderstand $Z_L$ auf den Wert $\tilde{Z}_L$ transformiert, dann ändert sich das Feldbild deutlich. Dazu ist es günstig, wenn $Z1$ entweder größer als beide oder kleiner als beide anderen Leitungswellenwiderstände ist. Dies wird nachfolgend anhand von Figur 9 erläutert.

Figur 9 zeigt zum Vergleich eine Figur 8 entsprechende graphische Darstellung des Ergebnisses einer weiteren Simulationsrechnung, welche die elektrische Feldstärke sowie magnetische Feldlinien bzw. Umrisslinien von magnetischen Flussröhren um einen Längsabschnitt von 0 bis 1 Meter eines langgestreckten elektrischen Leiters gemäß dem Ausführungsbeispiel der Figur 7 wiedergibt. Wie die Abbildung unten zeigt, weist eine solche Elektrodenleitung bei der Frequenz 64MHz auch eine starke Dämpfung auf. Die Energie am rechten Teil des Diagramms, also in Richtung zum distalen Ende der Leitung hin, ist deutlich geringer als bei der Elektrodenleitung nach dem Stand der Technik, deren Werte in Figur8 dargestellt sind.

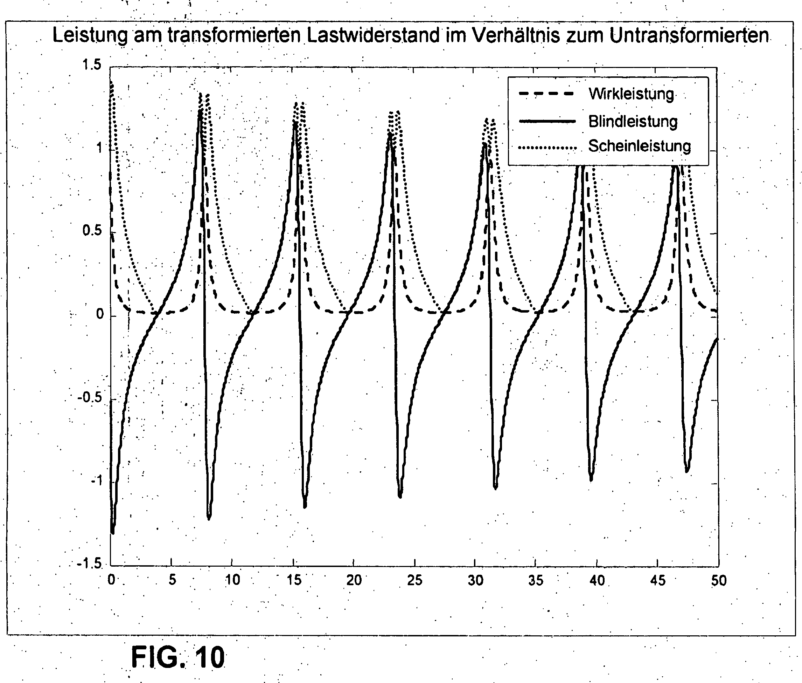

Zur weiteren Veranschaulichung der Transformationseigenschaften der Leistung ist in den Diagrammen der Figuren 10 bis 12 zunächst die Leistung an der Elektrodenspitze für ein Ausführungsbeispiel aufgezeichnet, bei dem zwischen einem Elektrodenpol in Form einer Elektrodenspitze und der Funktionsleitung ein kurzes, schwach bedämpftes Stück Leitung eingebaut ist. Auf der Abszisse der Diagramme der Figuren 10 bis 12 ist ein Längenparameter in Meter abgetragen, und an der Ordinate der Wert eines jeweiligen Leistungsparameters, wenn ein Stück Leitung mit einer an der Abszisse jeweils angegebenen Länge 1 zwischengesetzt ist, im Verhältnis zum betreffenden Leistungsparameter ohne Zwischenstück (1=0).

Figur 10 zeigt, dass über die Länge der Leitung periodische Schwankungen der Verhältnisse der Leistungsparameter Scheinleistung, Blindleistung und Wirkleistung auftreten. Über die Länge von 0 Zentimeter (proximales Ende) bis 20 Zentimeter ist über 7 Perioden eine Dämpfung erkennbar.

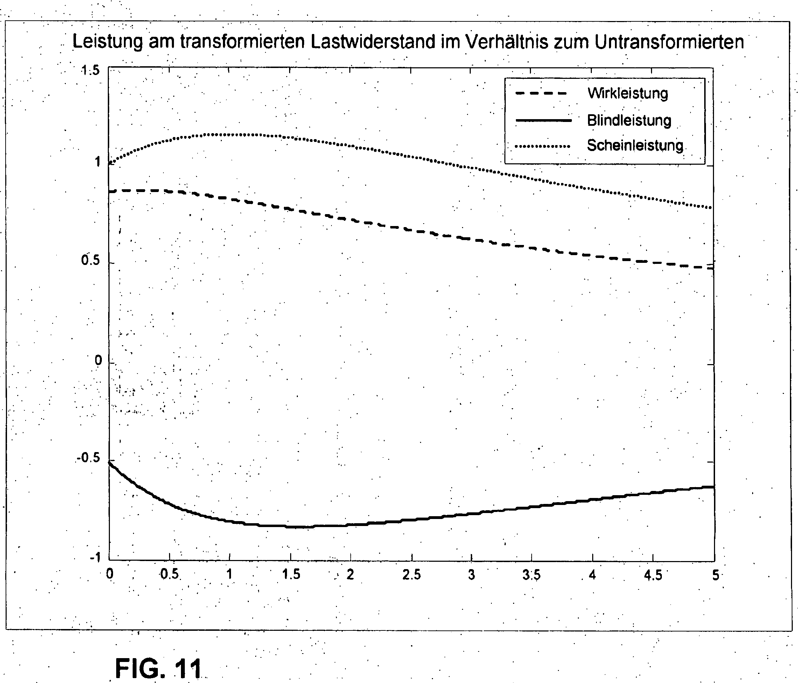

Im Diagramm der Figur 11 ist die Länge des eingefügten Leitungsstück auf der Abszisse abgetragen und das jeweilige Leistungsverhältnis am Lastwiderstand der Elektrodenspitze auf der Ordinate. Man erkennt an den zunächst über eine Länge von knapp 2 Millimetern ansteigenden Kurven der Wirkleistung und der Scheinleistung, dass das kurze Leitungsstück zur Reduzierung der Leistungsparameter länger als 2 Millimeter sein soll. Denn bis zu einer Länge von ca. 0,5 Millimeter steigt das Verhältnis der Wirkleistung mit dem zusätzlichen Leitungsstück zur Wirkleistung ohne das zusätzliche Leitungsstück an der Elektrodenspitze wegen der Transformationseigenschaften sogar. Bei einem 1cm langen Leitungsstück ist die Leistung in der Elektrodenspitze schon auf etwa die Hälfte reduziert.

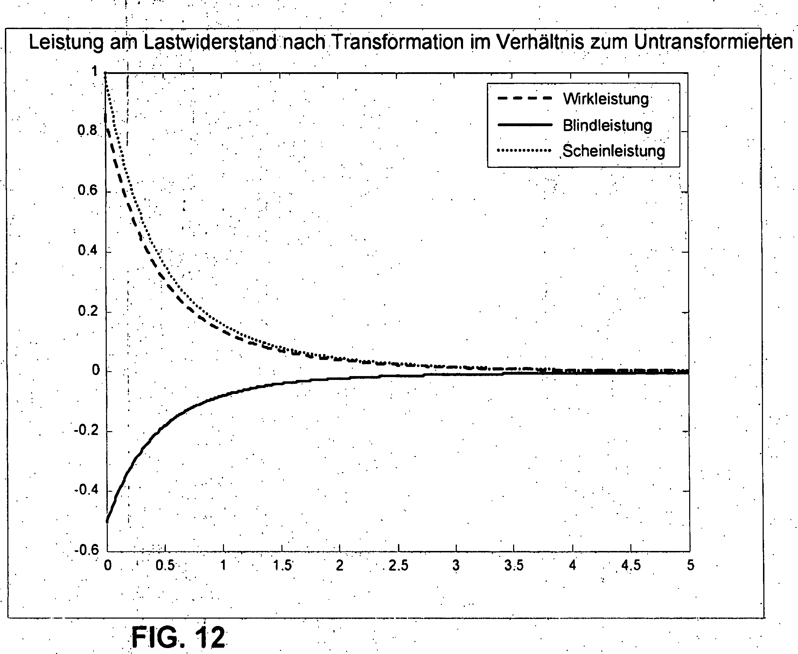

Günstiger sehen die Verhältnisse sogar noch aus, wenn man die Dämpfung der Leitung voll berücksichtigt, wie das Diagramm der Figur 12 zeigt. Bei voller Berücksichtigung der Dämpfung sinkt die Leistung am Lastwiderstand (Elektrodenspitze) schon nach 1 Millimeter Leitung auf unter die Hälfte des Wertes am Anfang der Leitung.

**Patentansprüche**

**1.** Temporär oder permanent implantierbares medizinisches Gerät (10) mit mindestens einem langgestreckten elektrischen Funktionsleiter (26), der durch ein Dielektrikum mit dem inneren Durchmesser $d_{iel}$ von umliegenden Material isoliert ist,

- der einen Elektrodenpol (22, 24) zur Abgabe von Therapiesignalen oder zur Erfassung von Diagnosesignalen aufweist, welcher im implantierten Zustand einen Lastwiderstand $Z_L$ für elektromagnetische Wellen darstellt,
- der zwischen seinem proximalen Ende und dem Elektrodenpol (22, 24) mindestens einen ersten Längsabschnitt (L1) eines ersten Leitungs-Wellenwiderstandes $Z_0$ für elektromagnetische Wellen aufweist, und
- der unmittelbar angrenzend an den ersten Längsabschnitt (L1) mindestens einen zweiten (L2), mindestens 0,25 innere Durchmesser ($d_{iel}$) langen, im Vergleich mit dem ersten Längsabschnitt kürzeren Längsabschnitt eines zweiten Leitungswellenwiderstandes $Z_1$ für elektromagnetische Wellen aufweist, wobei der zweite Leitungswellenwiderstand größer oder kleiner als der erste Leitungswellenwiderstand und größer oder kleiner ist als der Lastwiderstand und

der Funktionsleiter (26) einen Innenleiter und einen hohlzylindrischen Außenleiter aufweist, wobei der Innenleiter im zweiten Längsabschnitt (L2) eine vom Innenleiter im ersten Längsabschnitt (L1) abweichende geometrische Form hat, **dadurch gekennzeichnet, dass**
der Innenleiter im ersten Längsabschnitt (L1) hohlwendelförmig und im zweiten Längsabschnitt (L2) zylinderförmig ist, und
dass der zylinderförmige zweite Längsabschnitt (L2) des Innenleiters einen größeren Durchmesser als die Hohlwendel des Innenleiters im ersten Längsabschnitt (L1) aufweist.

2. Medizinisches Gerät nach einem der vorstehenden Ansprüche, bei dem ein Realteil des zweiten Leitungswellenwiderstands für elektromagnetische Radiofrequenz-Wellen um einen Faktor von mindestens 2 kleiner ist als ein Realteil des ersten Leitungswellenwiderstands.

3. Medizinisches Gerät nach einem der vorstehenden Ansprüche, bei dem im Funktionsleiter (26, 26') in Richtung zum distalen Ende hin an den zweiten Längsabschnitt (L2) ein weiterer erster Längsabschnitt (L1) des ersten Leitungswellenwiderstands $Z_0$ anschließt.

4. Medizinisches Gerät nach einem der vorstehenden Ansprüche, bei dem der zweite Längsabschnitt (L2) länger als 2 Millimeter ist.

5. Medizinisches Gerät nach einem der vorstehenden Ansprüche, bei dem der zweite Längsabschnitt (L2) kürzer als 20 Millimeter ist.

6. Medizinisches Gerät nach einem der vorstehenden Ansprüche, bei dem der zweite Längsabschnitt (L2) maximal 10 Millimeter lang ist.

7. Medizinisches Gerät nach einem der vorstehenden Ansprüche, bei dem der Innenleiter im ersten Längsabschnitt (L1) des elektrischen Leiters (20) hohlwendelförmig und im zweiten Längsabschnitt (L2) zylinderförmig ist.

8. Medizinisches Gerät nach Anspruch 7, bei dem der zylinderförmige Innenleiter im zweiten Längsabschnitt (L2) vom Außenleiter durch ein Dielektrikum von mindestens 5 Mikrometer Dicke isoliert ist.

9. Medizinisches Gerät nach einem der vorstehenden Ansprüche, bei dem der Elektrodenpol eine Elektrodenspitze (22) am distalen Ende des elektrischen Leiters (20) aufweist.

10. Medizinisches Gerät nach Anspruch 8, bei dem die Elektrodenspitze (22) unmittelbar an den zweiten Längsabschnitt (L2) anschließt.

**Claims**

1. A temporarily or permanently implantable medical device (10) comprising at least one elongate electrical functional conductor (26), which is insulated by a dielectric with the inner diameter $d_{iel}$ from surrounding material,

- which has an electrode pole (22, 24) for delivering therapy signals or for detecting diagnosis signals, which electrode pole in the implanted state constitutes a load resistor $Z_L$ for electromagnetic waves,
- which has, between its proximal end and the electrode pole (22, 24), at least one first longitudinal portion (L1) of a first line characteristic impedance $Z_0$ for electromagnetic waves, and
- which, directly adjacently to the first longitudinal portion (L1), has at least one second (L2) longitudinal portion, which is at least 0.25 inner diameters ($d_{iel}$) long and is shorter compared to the first longitudinal portion and has a second line characteristic impedance $Z_1$ for electromagnetic waves, wherein the second line characteristic impedance is greater than or less than the first line characteristic impedance and is greater than or smaller than the load resistance, and the functional conductor (26) has an inner conductor and a hollow-cylindrical outer conductor, wherein the inner conductor has a geometric shape in the second longitudinal portion (L2) deviating from the inner conductor in the first longitudinal portion (L1),

**characterised in that** the inner conductor is shaped in the form of a hollow helix in the first longitudinal portion (L1) and is cylindrical in the second longitudinal portion (L2), and
**in that** the cylindrical second longitudinal portion (L2) of the inner conductor has a greater diameter than the hollow helix of the inner conductor in the first longitudinal portion (L1).

2. The medical device according to the preceding claim, in which a real part of the second line characteristic impedance for electromagnetic radio frequency waves is at least 2 times smaller than a real part of the first line characteristic impedance.

3. The medical device according to any one of the preceding claims, in which a further first longitudinal portion (L1) of the first line characteristic impedance $Z_0$ adjoins the second longitudinal portion (L2) in the functional conductor (26, 26') in the direction towards the distal end.

4. The medical device according to any one of the preceding claims, in which the second longitudinal portion (L2) is longer than 2 millimetres.

5. The medical device according to any one of the preceding claims, in which the second longitudinal portion (L2) is shorter than 20 millimetres.

6. The medical device according to any one of the preceding claims, in which the second longitudinal portion (L2) is at most 10 millimetres long.

7. The medical device according to any one of the preceding claims, in which the inner conductor is shaped in the form of a hollow helix in the first longitudinal portion (L1) of the electrical conductor (20) and is cylindrical in the second longitudinal portion (L2).

8. The medical device according to claim 7, in which the cylindrical inner conductor is insulated in the second longitudinal portion (L2) from the outer conductor by a dielectric at least 5 micrometres thick.

9. The medical device according to any one of the preceding claims, in which the electrode pole has an electrode tip (22) at the distal end of the electrical conductor (20).

10. The medical device according to claim 8, in which the electrode tip (22) directly adjoins the second longitudinal portion (L2).

**Revendications**

1. Appareil médical (10) implantable de manière temporaire ou permanente avec au moins un conducteur fonctionnel (26) électrique allongé, qui est isolé du matériau environnant par un diélectrique avec le diamètre intérieur $d_{iel}$,

- qui présente un pôle d'électrode (22, 24) pour la délivrance de signaux thérapeutiques ou pour la détection de signaux de diagnostic, lequel représente une résistance de charge $Z_L$ pour des ondes électromagnétiques à l'état implanté,
- qui présente, entre son extrémité proximale et le pôle d'électrode (22, 24), au moins un premier segment longitudinal (L1) d'une première impédance de ligne $Z_0$ pour des ondes électromagnétiques, et
- qui présente, immédiatement adjacent au premier segment longitudinal (L1), au moins un deuxième segment (L2) longitudinal, plus court comparativement au premier segment longitudinal d'une deuxième impédance de ligne $Z_1$ pour les ondes électromagnétiques, long d'au moins 0,25 fois le diamètre intérieur ($d_{iel}$), où la deuxième impédance de ligne est supérieure ou inférieure à la première impédance de ligne et supérieure ou inférieure à la résistance de charge,

le conducteur fonctionnel (126) présente un conducteur interne et un conducteur externe en cylindre creux, où le conducteur interne dans le deuxième segment longitudinal (L2) a une forme géométrique dérivant de celle du conducteur interne dans le premier segment longitudinal (L1),

**caractérisé en ce que**

le conducteur interne est de forme hélicoïdale creuse dans le premier segment longitudinal (L1) et de forme cylindrique dans le deuxième segment longitudinal (L2), et

que le deuxième segment longitudinal (L2) en forme de cylindre du conducteur interne présente un diamètre plus grand que l'hélice creuse du conducteur interne dans le premier segment longitudinal (L1).

2. Appareil médical selon l'une des revendications précédentes, chez lequel la partie réelle de la deuxième impédance de ligne pour des fréquences radio électromagnétiques est inférieure d'un facteur d'au moins 2 par rapport à la partie réelle de la première impédance de ligne.

3. Appareil médical selon l'une des revendications précédentes, chez lequel, dans le conducteur fonctionnel (26, 26'), en direction de l'extrémité distale, un autre premier segment longitudinal (L1) de la première impédance de ligne $Z_0$ se raccorde sur le deuxième segment longitudinal (L2).

4. Appareil médical selon l'une des revendications précédentes, chez lequel le deuxième segment longitudinal (L2) est plus long que 2 millimètres.

5. Appareil médical selon l'une des revendications précédentes, chez lequel le deuxième segment longitudinal (L2) est plus court que 20 millimètres.

6. Appareil médical selon l'une des revendications précédentes, chez lequel le deuxième segment longitudinal (L2) a au maximum une longueur de 10 millimètres.

7. Appareil médical selon l'une des revendications précédentes, chez lequel le conducteur interne dans le premier segment longitudinal (L1) du conducteur électrique (20) est en forme d'hélice creuse et est de forme cylindrique dans le deuxième segment longitudinal (L2).

8. Appareil médical selon la revendication 7, chez lequel le conducteur interne de forme cylindrique dans le deuxième segment longitudinal (L2) est isolé du conducteur externe par un diélectrique d'une épaisseur d'au moins 5 micromètres.

9. Appareil médical selon l'une des revendications précédentes, chez lequel le pôle d'électrode présente une pointe d'électrode (22) à l'extrémité distale du conducteur électrique (20).

10. Appareil médical selon la revendication 8, chez lequel la pointe d'électrode (22) se raccorde immédiatement sur le deuxième segment longitudinal (L2).

FIG. 1

**26'**

$$Z_0$$

**22'**

$$U_0 \downarrow \qquad U_L \downarrow \qquad Z_L$$

**FIG. 2**

L

- Stand der Technik -

EP 2 465 571 B1

**26.1**

**26.2**

**26**

**22**

$$Z_0 \qquad I_E \qquad \qquad I_A$$

$$Z_1$$

$$U_0 \downarrow \qquad U_E \downarrow \qquad \qquad U_A \downarrow \qquad Z_L$$

**FIG. 3**

L1

L2

Gegenelektrode
(Schrittmacher)

FIG. 4

Gegenelektrode
(Schrittmacher)

FIG. 5

EP 2 465 571 B1

FIG. 6

FIG. 7

- Stand der Technik -

**FIG. 8**

FIG. 9

Leistung am transformierten Lastwiderstand im Verhältnis zum Untransformierten

- - - Wirkleistung
—— Blindleistung
······ Scheinleistung

FIG. 10

Leistung am transformierten Lastwiderstand im Verhältnis zum Untransformierten

Legend:
- – – – – Wirkleistung
- ——— Blindleistung
- ············ Scheinleistung

**FIG. 11**

EP 2 465 571 B1

Leistung am Lastwiderstand nach Transformation im Verhältnis zum Untransformierten

Wirkleistung
Blindleistung
Scheinleistung

FIG. 12

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007047966 A2